(19) Europäisches Patentamt
European Patent Office
Office européen des brevets

(11) **EP 1 798 291 B1**

(12) **EUROPEAN PATENT SPECIFICATION**

(45) Date of publication and mention
of the grant of the patent:
**18.05.2011 Bulletin 2011/20**

(51) Int Cl.:
*C12Q 1/68* (2006.01)

(21) Application number: **05027186.5**

(22) Date of filing: **13.12.2005**

(54) **Methods and compositions for assaying mutations and/or large scale alterations in nucleic acids and their uses in diagnosis of genetic diseases and cancers**

Verfahren für die Detektion von Mutationen in Nukleinsaüren, und die Anwendung in der Diagnose von genetischen Krankheiten und Krebs

Méthodes pour la détection de mutations dans les acides nucléiques, et leurs utilisation dans le diagnostique des maladies genétiques et des cancers

(84) Designated Contracting States:
**AT BE BG CH CY CZ DE DK EE ES FI FR GB GR HU IE IS IT LI LT LU LV MC NL PL PT RO SE SI SK TR**

(43) Date of publication of application:
**20.06.2007 Bulletin 2007/25**

(73) Proprietors:
• **Institut Curie**
**75248 Paris Cedex 05 (FR)**
• **Centre National de la Recherche Scientifique**
**75794 Paris Cedex 16 (FR)**

(72) Inventors:
• **Viovy, Jean-Louis**
**75013 Paris (FR)**
• **Houdayer, Claude**
**93310 Le Pre Saint-Gervais (FR)**
• **Stoppa-Lyonnet, Dominique**
**75007 Paris (FR)**
• **Weber, Jérémie**
**75020 Paris (FR)**

(74) Representative: **Le Coupanec, Pascale A.M.P.**
**Nony & Associés**
**3, rue de Penthièvre**
**75008 Paris (FR)**

(56) References cited:
WO-A-00/11221          WO-A-00/34652
WO-A-01/34839          WO-A-99/23257
US-A1- 2003 144 500

• **PALAIS ET AL: "Quantitative heteroduplex analysis for single nucleotide polymorphism genotyping" ANALYTICAL BIOCHEMISTRY, ACADEMIC PRESS, NEW YORK, NY, US, vol. 346, no. 1, 1 November 2005 (2005-11-01), pages 167-175, XP005109918 ISSN: 0003-2697**
• **WIKIPEDIA: 'Mutation' INTERNET CITATION, [Online] Retrieved from the Internet: <URL: en.wikipedia.org/wiki/Mutation> [retrieved on 2008-05-19]**
• **STRACHAN; READ: 'Human Molecular Genetics 2', vol. 14.2, 1999, BIOS SCIENTIFIC PUBLISHERS**

**Description**

Recent developments in genomics have raised considerable hopes for improvements in human health and biotechnology.

In medicine, for instance, the understanding and diagnosis of genetic diseases and cancers, or the study of infectious organisms, rely more and more on analysis of DNA and nucleic acids.

Biotechnology is also more and more dependent upon molecular genetic and nucleic acids high-throughput analysis. In particular, mapping of genetic differences between individuals is of growing importance for forensic investigations, medical applications, biotechnology and food industry.

For example, detecting mutations leading to abnormal proteins can be essential for identifying the genetic origin of a disease. A number of inherited pathological conditions may be diagnosed before onset of symptoms, using methods for structural analyses of DNA. In cancer research, for example, the search of mutations in BRCA1 and BRCA2 genes, recognized to lead to strong increase in the probability of developing breast cancer, is now performed on a large scale. The gene APC is also known to lead to strong predisposition to colorectal cancer. Genetic screening can also be performed at an early age, or even in utero, for numerous heritable diseases. At present, 700 genetic diseases have been identified among which are thalassemia and myopathy.

The identification and detailed analysis of acquired genetic disorders, such as those arising in particular in cancer, is also raising the hopes for more efficient and personalized treatments, by means of "genetic mapping" of tumors. Large scale genetic screening of mutations and genetic variability, also called "genotyping", is also of paramount importance for determining correlations between diseases and genes, in order to find new targets for therapy in a pharmacogenomic approach.

Genetic screening can also be used for detecting pathogens, including identification of specific pathogenic varieties in medicine, food industry, veterinary or bioterrorism applications. For example, in food industry, the detection of genetically modified organism "GMO" in starting material or foodstuff is an increasing concern.

Mutations implicated in diseases mechanisms can be point mutation (single base substitution or small insertion or deletion of one or several tenth of base pairs) or large chromosomic rearrangments.

A need exists, therefore for a methodology to detect mutations concerning a gene relative to a non pathological state (wild type), or to identify different alleles of a gene, in an accurate, reproducible and reliable manner.

DNA molecules are linear polymers of subunits called nucleotides. Each nucleotide comprises a common cyclic sugar molecule, which is linked by phosphate group to the sugar of the adjoining nucleotide, and one of the different cyclic substituants called bases. The combination of the sugar and base is called a nucleoside. The four bases commonly found in DNAs from natural sources are adenine, guanine, cytosine and thymidine, hereinafter referred to as A, G, C and T, respectively. The linear sequence of these bases in the DNA of an individual is its "genome". It involves coding regions, which bear the information for synthesis of proteins, regions for regulation of gene expression, and so called "non-coding" regions, the role of which being not fully understood.

In double-stranded DNA, the form adopted by DNA in the chromosomes of all cellular organisms, the two DNA strands are entwined in a precise helical configuration with the bases oriented inward, allowing interactions between bases from opposing strands. The two strands are held together in precise alignment mainly by hydrogen bonds which are permitted between bases by a complementarity of structures of specific pairs of bases. This structural complementarity is determined by the chemical natures and locations of substituents on each of the bases, leading in particular to a definite number and orientation of hydrogen bonds. Thus, in double-stranded DNA, normally each A on one strand has an attractive interaction with a T from the opposing strand, involving two hydrogen bonds, and each G has an attractive interaction with an opposing C involving three hydrogen bonds. In principle, they insure that DNA molecules are replicated and precise copies are passed on to the cell descendants during cell reproduction (mitosis), or to the offspring of the individual, when replication concerns the gametes (meiosis).

Occasionally, an incorrect base pairing may occur during replication, which, after further replication of the new strand, results in a double-stranded DNA offspring with a sequence containing a heritable base difference, like deletion, insertion or substitution of 1 or more bases from that of the parent DNA molecule. Such heritable changes are called genetic mutations, or more particularly in the present case, "point mutations". Mapping of genetic mutations involves both the detection of sequence differences between DNA molecules comprising substantially identical (i.e., homologous) base sequences, and also the physical localization of those differences within some subset of the sequences in the molecules being compared. Variations in the DNA sequence may also affect non-coding regions. In particular, high variability such as short tandem repeats (STR) or Single nucleotide polymorphism (SNP) may exist in non-coding regions, and are very useful in genotyping.

Detecting point mutations, and in particular substitutions, is particularly challenging, because they are very localized, and in many occurrences their position cannot be known in advance. Actually, for many mutations associated with diseases, the exact location of a mutation is not known a priori, and the whole coding sequence of the gene (generally representing several thousands or tens of thousands of bases) must be screened completely. Occasionally, some large scale alteration(s) may also appear. Recent research has revealed a group of diseases that are due to the rearrangement of specific genomic segments from a few kilobases to several megabases in length. As rearrangements concern generally more than 1 kilobase, techniques based on standard PCR are not suitable for detection of such mutations.

The most prominent technologies at present to detect point mutations are sequencing and Denaturing HPLC.

In the approach "Direct sequencing" the whole gene is sequenced totally for each patient, a powerful but costly method. In addition, due to the presence of heterozygoty, mutated patient sequences are not « pure », and the interpretation of data is not as straightforward as for conventional sequencing. Most importantly, this method is long and costly, since the whole gene and the flanking regions must be entirely sequenced for each patient or individual to screen. The deletion or duplication of a gene or a part of a gene can not be detected with this method.

"Chromatography in a gradient of denaturing conditions" (DHPLC) is based on the separation of heteroduplexes from homoduplexes (Oefner PJ, Underhill PA. 1995. Comparative DNA sequencing by denaturing high-performance liquide chromatography (DHPLC). Am. J. Hum. Genet. 57:S: A266.). When a DNA fragment including the location of the gene is amplified by PCR, both genes are amplified. When the amplified DNA is denatured and then renatured slowly, 4 different types of duplex DNA may be obtained, i.e.: two homoduplexes, corresponding to the DNA of the two intial alleles (the normal one and the mutated one), and two heteroduplexes, mixing one strand from one allele and the (almost) complementary strand from the other allele. These heteroduplexes contain, at the location of the mutation, a mismatch "bubble". These mismatches "bubbles" may be searched by DHPLC. The renaturated (homoduplexes and, when relevant, heteroduplexes) DNA are adsorbed on a HPLC column, and the denaturation of DNA (which occurs slightly earlier for heteroduplexes) leads to the release of the DNA, and to detection at the output of the column (see e.g. Wagner, T., Stoppa-Lyonnet, D., Fleischmann,E., Muhr, D., Pages, S., Sandberg, T., Caux, V., Moeslinger, R., Langbauer, G., Borg, A., Oefner, P., Genomics, 1999, 62, 369-376). However, the whole process of DHPLC is long when large genes have to be screened, because of the sequential nature of HPLC, which can analyse only one sample at a time. Furthermore, this method does not allowed the detection of large scale alterations

The others methods used for the detection of point mutations such are single strand conformation polymorphysm (SSCP) (Orita M, Iwahana H, Kanazawa H, Hayashi K, Sekiya T. 1989. Detection of polymorphisms of human DNA by gel electrophoresis as single-strand conformation polymorphisms. Proc Natl Acad Sci U S A 86(8):2766-70), chemical cleavage (Cotton RG, Rodrigues NR, Campbell RD. 1988. Reactivity of cytosine and thymine in single-base-pair mismatches with hydroxylamine and osmium tetroxide and its application to the study of mutations. Proc Natl Acad Sci U S A 85(12):4397-401) and direct sequencing are not also able to detect large rearrangements.

Regarding large scale alterations, the most prominent technologies at present, are based on multiplexed semi-quantitative PCR.

PCR amplification may be decomposed in several phases. During the first one (dNTPs and probes are in excess, DNA polymerase activity is high) amplification is exponential with an amplification rate close to $2^n$. Then this amplification rate decreases, the creation of new DNA saturates and reaches a plateau. In order to remain in the exponential part of the PCR, one has to limit the number of PCR cycles, i.e. performing the PCR in non-saturating conditions. Overall, the total DNA content follows a sigmoidal curve.

If the number of PCR cycles is limited to remain in the fast increasing area of this sigmoid, the final number of copies of a fragment after a given number of amplification cycles depends strongly on the initial number of copies. More specifically, it is roughly proportional to the initial number of copies. Two or more DNA fragments can be simultaneously amplified with PCR. This is called multiplexed PCR. By comparing the final number of copies of these two fragments in the final sample, it is thus possible to relatively quantify the initial DNA concentration

By comparing the ratio of band intensities between at least two predefined peaks it is possible to determine the presence of a large duplication or deletion. Indeed a normal patient will have two normal copies of the gene or part of the gene which will lead to a certain ratio. A patient with a deletion of the gene or part of the gene will have only one normal copy of a gene or part of the gene, which will lead to a ratio two times lower than the normal patient. Finally a patient with a duplication of the gene or part of the gene will have three normal copies of the gene or part of the gene, which will then lead to a ratio 1.5 times higher than the normal patient. If there are multiple duplications of the gene, the ratio can be higher than 1.5.

WO0134839 describes a method for detecting point mutations and deletions in several genes or fragments simultaneously. A multiplex PCR is done with primers flanking the regions of interest on a sample nucleic acid comprising the "test nucleic acid" and a standard nucleic acid. These nucleic acid fragments are denatured and reannealed to form homo- and heteroduplexes, possibly between the standard and the test nucleic acid. The reannealed product is analysed for determining the duplex form. The method is suitable for obtaining relative quantitative data by comparing one PCR products signal to the other. The data obtained are compared with a standard nucleic acid without any of the mutations

"Multiplex ligation-dependent probe amplification" (MLPA) (Schouten JP, McElgunn CJ, Waaijer R, Zwijnenburg D, Diepvens F, Pals G. 2002. Relative quantification of 40 nucleic acid sequences by multiplex ligation-dependent probe amplification. Nucleic Acids Res 30(12):e57) is based on semi-quantitative PCR of specific DNA probes. Couples of DNA probes that hybridize to two adjacent sites are used. One of the probes has a size dependant sequence that does not hybridize on DNA and that is depending on the studied part of the gene. One of the probes has a specific sequence at its 3' end that is complementary to a PCR primer. The other probe has a specific sequence at its 5' end that is complementary to another PCR primer. Several couples of probes can be used simultaneously. After probe hybridization a specific enzyme, a ligase, is used in order to make a ligation between the two adjacent probes. A part of a gene with no duplication or deletion will lead to the formation of two ligated probe couples. A part of a gene with a duplication will lead to the formation of three ligated probe couples. A part of a gene with a deletion will lead to the formation of one ligated probe couple. Probes are then amplified using a single set of primers (all ligated probes have the same PCR primer hybridization sequence at their 3' end and all ligated probes have the same PCR primer hybridization sequence at their 5' end). Amplified fragments are then separated using gel or capillary electrophoresis and up to 40 different DNA regions can then be scanned in the same reaction. This method does not allow the detection of point mutations.

"Quantitative multiplex PCR of short fluorescent fragments" (QMPSF) (Casilli F, Di Rocco ZC, Gad S, Tournier I, Stoppa-Lyonnet D, Frebourg T, Tosi M. 2002. Rapid detection of novel BRCA1 rearrangements in high-risk breast-ovarian cancer families using multiplex PCR of short fluorescent fragments. Hum Mutat 20(3):218-26) is based on the use of specific PCR primers in order to allow the simultaneous amplification of more than 10 DNA regions. All forward primers carry a specific extension at their 3' end. All reverse primer carry a specific extension at their 5' end. These extensions of 16 nucleotides are very rare sequences that are not complementary of the studied DNA. They allow from the 2nd PCR cycle to have a very homogenous annealing temperature. The use of these specific primers reduces the efforts invested in adjusting relative primer concentrations, in order to achieve homogeneous fluorescent peak levels. This method allows the detection of small insertions and deletions.

These methods are efficient for the detection of large scale alterations, involving entire genes, or sequences differences between the normal and mutated DNA of several tens to several thousands of bases. However, they provide no information on point mutations, in particular substitution. When a patient or more generally an organism is suspected to bear a mutation, one does not know a priori if this mutation involves relatively large scale or local mutations. At present, these two types of mutations are searched for in parallel, using two different methodologies. This implies a duplication of effort, as well as an increase of costs.

Other method used to detect large scale alterations such as real time PCR or Multiplex liquid chromatography (MP/LC) (Dehainault C, Lauge A, Caux-Moncoutier V, Pages-Berhouet S, Doz F, Desjardins L, Couturier J, Gauthier-Villars M, Stoppa-Lyonnet D, Houdayer C. 2004. Multiplex PCR/liquid chromatography assay for detection of gene rearrangements: application to RB1 gene. Nucleic Acids Res 32(18):e139) are also not able to detect single base substitutions and are not very reliable for small insertions and deletions.

Accordingly, there is still a need for a simple and very specific method which may allow to detect simultaneously mutations as different as point mutation(s) and large scale alteration(s) in nucleic acids such as genomic DNA and which may be performed quickly, accurately and easily with minimal operator skills, as well as with a reduced amount of costs.

It is precisely an object of the invention to allow the simultaneous search of large scale and local mutations, and thus a simplification and an acceleration of the mutation search effort.

More precisely, it is an object of the present invention to propose a method for detecting point mutation(s) and/or large scale alteration(s) concerning at least one nucleic acid fragment said "first nucleic acid fragment(s)", comprising at least the steps consisting in:

a/ providing a sample liable to contain said first nucleic acid fragment and at least a second nucleic acid fragment distinguishable from the first nucleic acid fragment and acting as a quantitative reference,

b/ denaturating said nucleic acid fragments and reannealing them in conditions suitable for obtaining a product containing homoduplexes and possible heteroduplexes,

c/ conducting on said reanneled product an analytical method suitable for obtaining at least signal(s) discriminating the existing duplex form(s) of the first nucleic acid fragment and relative quantitative data on said first nucleic acid fragment by comparing the intensity of its signal to the intensity of the signal obtained from the second reference nucleic acid fragment and

d/ comparing the relative quantitative data obtained in step c) to a control relative quantitative data expected for a first nucleic acid fragment in which no large scale alteration is present.

The obtaining of the said possible heteroduplexes at step b) may occur if the first nucleic acid fragment is present in said sample in at least two forms with different sequences.

In a first embodiment, the method according to the instant invention may comprise at least an additional step e) involving analysis of the shape of the signal(s) from the first nucleic fragment obtained in step c).

This additional analysis may be performed by comparing the shape of the signal(s) from the first nucleic acid fragment to the shape of the signal obtained by applying steps a) to c) to a form of said first nucleic acid fragment being equivalent to a non mutated form of said first nucleic acid fragment(s).

According to one embodiment, said first nucleic acid fragment(s) and said second reference nucleic acid fragment may be obtained by applying restriction enzymes to a DNA-containing sample.

In another embodiment, said first nucleic acid fragment(s) and said second reference nucleic acid fragment may be obtained by a nucleic acid amplification method. A nucleic acid amplification method may be, for example, NASBA, Rolling Circle Amplification (RCA) or polymerisation chain reaction (PCR).

According to another of its aspects, the invention is related to a method for the diagnostic of a predisposition to cancer, comprising a method according to the invention.

According to another of its aspects, the invention is related to method for cancer diagnosis, comprising a method in accordance with the invention.

According to another of its aspects, the invention is related to a method for the diagnostic of a genetic disease, comprising a method in accordance with the invention.

According to another of its aspects, the invention is related to a method for discovering new targets for therapy or screening efficiency of a therapy, comprising a method in accordance with the invention.

According to another of its aspects, the invention is related to a method for discovering new biomarkers for a disease or a pathological condition, comprising a method in accordance with the invention.

## Definition

According to the invention:

- the expression "nucleic acid" intends to encompass any synthetic or natural DNA, RNA, nucleic acids analogs such as PNA (peptide nucleic acids), LNA, thiolated DNA, etc...
- the expression "nucleic acid fragment" intends to mean a stretch of nucleic acid comprised (inclusively) between two predefined flanking sequences. Typically, a nucleic acid fragment within the invention may have a length ranging from 50 bp to 50 000 bp, and preferably from 100 bp to 1000 bp. A nucleic acid fragment A is said "equivalent" to a nucleic acid fragment B, if it is comprised between the same two flanking sequences, but may have a sequence equal or different from this of fragment B.
- the expression "flanking sequences" intends to mean a pair of sequences along the genome of an organism or individual, that defines the two sides of a sequence inside this genome. The most common flanking sequence that may be used within the invention, may be a pair of primer sequences. More particularly, it may be an oligonucleotide with a predefined sequence, able to hybridize on a single strand nucleic acid and to initiate replication on said single strand nucleic acid, when put in presence of nucleotides and a polymerase. Another example of a pair of flanking sequences may be given by a sequence of two distinct and consecutive restriction sites along a DNA molecule, at which a restriction enzyme will cut the DNA. Using restriction enzymes as flanking sequence, in opposition to PCR primer pairs, may have the advantage of avoiding the need of amplifying DNA,
- the expression "primer sequence pair" intends to mean a pair of primer sequences able to hybridize onto two complementary strands of a DNA and lead to amplification by polymerase chain reaction,
- the term "signal" intends to mean a peak or an ensemble of peaks corresponding to one nucleic acid fragment in a profile data resulting from the application of an analytical method to a reanneled product obtained in step c), d) and/or e) of a method according to the instant invention,
- the expression "signal intensity" intends to means a quantitative determination of a quantity of material corresponding to a given peak or signal,
- the expression "nucleic acid amplification" intends to means any method able to generate, in a sample, multiples copies of at least one nucleic acid fragment comprised between two predefined sequences. Preferably, the number of copies generated in the final product may be a function of the number of copies initially present in the sample,
- the term "first nucleic acid fragment(s)", will designate any of a multiplicity of nucleic acid fragments in a sample, regarding which one is searching for a mutation. "First" is thus used as a generic expression do differenciate the fragment on which one searches such mutation, from the "second" nucleic acid fragment, which is used as a reference in the same sample,
- the expression "form equivalent to a non mutated form of a first nucleic acid fragment" intends to cover any non mutated wild type forms of said first nucleic acid fragment including forms having a polymorphism, i.e. having not any mutation within the meaning of the invention i.e. implicated in diseases mechanisms.

## DETAILED DISCLOSURE OF THE INVENTION

The method of the instant invention is particularly advantageous since it allows to proceed in a single run and on the same first nucleic acid fragment(s), to two distinct analysis.

Furthermore, the instant invention is not limited to the search in a single run of point mutation(s) and large scale alteration(s) on a single first nucleic acid fragment.

It may be applied to a sample comprising several "first acid nucleic fragments" in a single run of the separation method.

In effect, the method according to the invention may be efficiently applied to the detection of point mutation(s) and /or large scale alteration(s) concerning more than 1, in particular more than 2, in particular more than 5 and more particularly on a large number of different nucleic acid fragments, named "first nucleic acid fragments" belonging or not to the same gene.

Thus, the invention may offer the advantageous possibility of performing such search on several nucleic acid fragments following one single separation step by an analytical method. This advantageous embodiment of the invention is called in the following "multiplexing".

In case where several "first" nucleic acid fragments are present in one sample within the invention, these nucleic acid fragments must be distinguishable.

As stated previously, one of both analysis may be directed to the detection of point mutation(s).

More particularly, the presence of different alleles in a sample, leading to an heteroduplex, may be used in the purpose of determining the presence of a point mutation. It may be a way to recognize the presence of a mutation, including a single substitution, on a fragment on which an heteroduplex is formed. This means that the sample may contain a nucleic acid fragment simultaneously in a mutated and a non-mutated form.

For example, if the organism from which the sample is obtained is heterozygote, and contains at least one copy of a mutated gene, and at least one copy of a corresponding normal gene, an heteroduplex may naturally be formed during reannealing, without addition of any other nucleic acid. This case is very common and may correspond, for instance, to all cases in which patients are heterozygous for a searched mutation, as occuring as a matter of example, and non exhaustively, for mutations along the BRCA1 or BRCA2 genes, which predispose to breast cancer.

If an organism, tissue, or more generally a biological material to be screened is homozygote for the mutation, heteroduplex may still be obtained as a signature of the mutation, by adding into the sample a reference nucleic acid, known to be non-mutated in the fragment under consideration. In this case, preferably, the quantity of nucleic acid corresponding to the reference, non-mutated fragment added to the sample, is to be adapted so that the number of copies of the reference non-mutated fragment is comprised between 0.1 times and 10 times, preferably between 0.5 times and 2 times, and yet more preferably equal to, the number of copies of the fragment under consideration.

It is worth to notice that one of the advantages of the invention, as compared e.g. to direct sequencing or to DHPLC, is that it is less sensitive to differences in the concentrations of normal and mutated nucleic acid. Thus it increases the robustness of protocols. Typically, a method of the invention may be able to detect a presence of a mutated fragment at a concentration as small as 10% or less, or as large as 90% or more

Advantageously, the presence of point mutation(s) may be also detected by analyzing shape of the signal obtained in step c) for the first nucleic acid fragment(s).

More particularly, this analysis may be performed by comparing shape of the signal obtained for said first nucleic acid fragment(s) to the shape of the signal obtained for a form of the first fragment(s) equivalent to a non mutated form of said first nucleic acid fragment(s) and which have been obtained in similar analytical conditions as disclosed more precisely hereinafter.

In a preferred embodiment, multiplexing may be achieved by combining in a same run fragments with different lengths. Since the homoduplex and heteroduplexes generally have close mobilities, several combinations of homoduplexes and heteroduplexes may be separated and identified in a single run by mixing first nucleic acid fragments fragments with sizes sufficiently different between themselves, and sufficiently different from the second reference nucleic acid fragment(s).

The other analysis, directed to the detection of large scale alteration(s) like deletion(s) and/or duplication(s) is based on a quantitative analytical method and the use, in combination to the first nucleic acid fragment(s) to analyze, of a reference nucleic acid fragment, also called second nucleic acid fragment, distinguishable from the first one(s). This method comprises firstly an evaluation of quantitative data relative to a first nucleic acid fragment by referring to said reference nucleic acid fragment and secondary a comparison of the so-obtained relative quantitative data to a quantitative data expected, in the same conditions of analysis, for a first nucleic acid fragment(s) in which no large scale mutation involving said first nucleic acid fragment is present.

Regarding the reference nucleic acid fragment, it has to be distinguishable from the first nucleic acid fragment(s) to analyze.

According to one embodiment, second nucleic acid fragment and some or all of the first nucleic acid fragment(s) may differ by at least their respective length. In particular, they may differ by at least 1, in particular by at least 3, in particular by at least 5, in particular by at least 10, in particular by at least 15, in particular by at least 20, in particular by at least 30 bases, and more particularly by at least 100 bases.

In all cases, the first and second nucleic acid fragments in the sample must be distinguishable. Typically, if the analytical method is electrophoresis, the fragments must differ in size by a factor between 10 and 100 bp, preferably between 20 and 100 bp. If the analytical method is Mass spectrometry, however, is may be sufficient to have fragments that differ in size by a factor between 2 and 100, since this method has a higher resolution.

A similar difference of length may applied between the different said first nucleic acid fragments when multiplexing, as detailed thereafter, is performed.

For technical reasons appreciated by the skilled person, the difference of length may be not greater 1000 bases.

According to a second embodiment, the second nucleic acid fragment and some or all first nucleic acid fragment(s) may be made distinguishable from each other by at least distinct markers. These markers may be fluorescent or isotopic, and may be for example, primers fluorescently or isotopically labelled.

In an embodiment, which may be combined with the previous one, multiplexing may be performed by preparing different samples bearing tags with fluorescence at different wavelength, using e.g. PCR with different fluorescently labeled primers. The amplification of the different samples may be performed in a single reaction, and the mixture may be analysed in a single electrophoresis run. Several capillary electrophoresis machines, designed for DNA sequencing or fragment analysis, such as the ABI 310, 3100, 3700, or the Amersham "Megabace", may analyse simultaneously products with different fluorescence emission wavelength.

Using different fluorescence tags may also be used if the analytical method is a chromatographic method. Finally, tagging by different isotopes may be used if the analytical method is Mass Spectrometry.

Naturally both previous embodiments proposed for distinguishing first and second nucleic acid fragment(s) i.e. by respective lengths or distinct markers may be combined.

The second reference nucleic acid fragment may be naturally present in the sample and in particular the biological sample liable to contain the first nucleic acid fragment(s) to analyze. Naturally, it will be selected in order to comply with the previous requirements for distinguishing it from the fragment(s) to analyze.

According to an embodiment, the second reference nucleic acid fragment and the first nucleic acid fragment(s) may be derived from two distinct genes.

According to an embodiment, the second reference nucleic acid fragment and the first nucleic acid fragment(s) may be derived from the same gene.

According to another embodiment, a reference nucleic acid fragment may be incorporated, into the sample liable to contain said first nucleic acid fragment(s). In such a case, it may be introduced in a quantity predefined in regard of the total quantity of nucleic acid present in the sample.

For example, to facilitate the quantitative comparison between a signal from said first nucleic acid fragment(s), and a signal from said second reference nucleic acid fragment, it may be preferable that the two signals have not too different amplitudes. For instance, it may be suitable that a signal from the second nucleic acid reference fragment to be comprised between 0.1 times and 10 times, preferably between 0.5 times and 2 times, the signal from said first nucleic acid under consideration. To achieve this aspect, one can make a measure of the total concentration c (e.g. in g/l) of the total nucleic acid quantity in the initial sample, e.g. by optical density at 264 nm.

For a sequence present in a genome as a single copy (which is generally the case for a coding sequence), the number of copies of this sequence per liter in the sample may be equal to the total nucleic acid concentration c, divided by the total molecular weight of the organism's genome. It is then easy to provide a second reference nucleic acid fragment in a comparable concentration. One particularly simple way to do so, may be to provide a second reference nucleic acid fragment by co-amplifying, with said first nucleic acid fragment, a second nucleic acid fragment on which no genetic alteration is known.

The use of such a second reference nucleic acid fragment may allow to obtain a first relative quantitative data for the first nucleic acid fragment(s) present in the analyzed sample. By comparing this first relative quantitative data to a relative control quantitative data, obtained for a first nucleic acid fragment(s) in which no large scale alteration is present in conditions of analysis representative of the conditions elected in the steps a) to d) for the analysis of corresponding first nucleic acid fragment(s), a conclusion on a possible presence of large scale alteration(s) concerning the analyzed first nucleic acid fragment(s) may be obtained.

This control relative quantitative data may be obtained, at the same time or not, than the relative quantitative data for the sample to be analyzed by just reproducing at least steps a) to d) on a control sample i.e. containing a first nucleic acid fragment(s) in which no large scale alteration is present and same second reference nucleic acid fragment. This control analysis may be performed simultaneously with the analysis of the sample, but it is not necessary.

Indeed, it may be an important advantage of the invention that the relative quantitative data may be reproducibly obtained relative to an internal second reference nucleic acid fragment, present in the sample in a ratio of concentration with regards to said first nucleic acid, between different experiments.

Thus, a control relative quantitative data may be obtained in a separate run, provided that the sample analysed in said separate run contains both the first nucleic acid fragment(s) and the second reference nucleic acid fragment, and that the ratios of concentrations between said first and said second nucleic acid fragments are the same in the sample to be analyzed and the control sample.

A control relative quantitative data may be obtained by reproducing exactly the conditions of a method of the invention as defined in steps a) to d) applied for analyzing a corresponding sample. However, it may also be obtained from equivalent or different conditions with the proviso that the conditions of the method as defined in steps a) to d) applied for analyzing the corresponding sample may be extrapolated based on these equivalent or different conditions.

In an embodiment, a control relative data may be a ratio of intensity of signal(s) from said first nucleic acid fragment(s), to intensity of signal from said second reference nucleic acid fragment.

Generally, one single second nucleic acid fragment may be used as a reference for several first nucleic acid fragments in a same sample.

However, in some cases several second nucleic acid fragments may be used.

Also, in some cases a "first" nucleic acid fragment, may be used as a reference for other(s) first nucleic acid fragment(s), provided that said first nucleic acid fragment used as a reference is not liable to be involved in a large scale mutation or otherwise, presents the same number of copies as the wild type.

This specific embodiment may be achieved, for instance, when separating in similar conditions numerous samples from different patients since the probability of occurrence of one specific mutation in the population is low. Then, most of the the quantitative ratios between signal intensities are invariable from one sample to the other, and the rare anomalies may be easily identified as a signature of a large scale mutation.

Typically, the method of the invention may be able to detect the presence of large scale alteration(s) in a mutated fragment at a concentration as small as 1%, or as large as 99%.

## ANALYTICAL METHOD

An analytical method used in the method according to the invention may be any method able to differentiate two duplex DNA molecules, as a function of their length, as a function of their conformation, or preferably both.

According to an embodiment, a separating analytical method suitable for the instant invention may be an electrophoretic, chromatographic or a mass spectrometric method.

Electrophoresis may be a particularly suitable method for this, because of its convenience of use, of its quantitative character, and of the high resolution it proposes in size and conformation discrimination.

Separation in "lab on chips" also called microfluidic systems may also be of high potential.

However, the analytical method may also be a mass spectrometry (MS) method. Detection of point mutations by mass spectrometry has already been proposed in Laken SJ, Jackson PE, Kinzler KW, Vogelstein B, Strickland PT, Groopman JD, Friesen MD. 1998. Genotyping by mass spectrometric analysis of short DNA fragments. Nat Biotechnol 16(13):1352-6, but the joint determination of point mutation(s) and large scale alteration(s) by MS was never proposed. If MS is used as the analytical method, it may be in general advantageous to use nucleic acid fragments smaller than for chromatography or electrophoresis, typically in the range of 30 bp to 500 bp, and preferably 50 bp to 250 bp.

In a preferred embodiment of the present invention, the analytical method may be an electrophoretic analysis, in particular comprising the use of a separation medium.

More particularly, the nucleic acid(s) having mismatch, in particular heteroduplex(es), may be detected by a capillary electrophoresis or multicapillary electrophoresis for instance in microchannels.

An analytical method of step c) of a method of the invention may be performed in non-denaturating conditions.

The invention may be particularly advantageous for capillary electrophoresis or multicapillary electrophoresis in microchannels for the following reasons.

It may improve significantly the sensitivity of electrophoretic analysis, especially for difficult detectable mismatches such as substitutions.

It may allow for a much faster separation than sequencing.

It may allow for multiplexing in different ways, thus increasing further the throughput.

The approach, termed "Electrophoretic Heteroduplex Analysis", (EHDA) directly measures, by electrophoresis in non-denaturating conditions, the difference in mobility induced by a « denaturation bubble » or « loop » associated with the mismatch appearing in heteroduplex pairs.

When a sample is separated by electrophoresis in non-denaturing conditions, the presence of a local "mismatch bubble" on the heteroduplex molecules leads to a difference in geometry of flexibility of the double strand, which leads to a difference in mobility, as compared to the homoduplexes. If a nucleic acid fragment to analyse presents a normal duplex fragment and a mutated fragment (i.e. if the organism from which the DNA is extracted is heterozygote for the corresponding DNA fragment), a multiplicity of peaks (two to four) will appear in the electrophoregram. These different peaks correspond to the same fragment in the sense of the invention, i.e. they are delimited by the same flanking sequences.

The power of the EHDA method relies mainly on the ability to detect the slight difference in migration velocity, due to the presence of mismatch bubble.

Different types of electrophoresis, slab gels electrophoresis, capillary electrophoresis, multicapillary electrophoresis, or multichannel electrophoresis, may be used. Multicapillary electrophoresis may be particularly privileged for high-throuhgput automated screening.

In an embodiment, multiplexing may be achieved by combining in a same run fragments with different lengths. Since the homoduplex and heteroduplexes generally have close mobilities, several combinations of homoduplexes and heteroduplexes may be separated and identified in a single run, by mixing fragments with sufficiently different sizes. Typically, the fragments must differ in size by a factor between 10 and 100 bp, preferably between 20 and 100 bp.

Multiplexing may be achieved by performing several sequential injections of different samples. The principle may the same as for the separation of fragments of different sizes: it may use the fact that, in contrast with e.g. sequencing, electrophoretic analysis of a single fragment may only use a very limited part of the separation window offered by electrophoresis, so that several samples, with start time suitable shifted, may be separated in a single run.

In another embodiment, a separation of the heteroduplex fragments may be performed by DGGE, in the presence of a composition as described in the invention, or by DHPLC in the presence of the same type of composition.

### Separation medium

Different types of separation medium or matrices may be use for separation according to the invention.

Indeed any matrix or separation medium able to separate nucleic acids by size may be used. For slab gel electrophoresis, the most useful matrices may be polyacrylamide gels, but others may be used, such as, given a non-exhaustive list of examples, agarose, or other matrices reviewed in e.g. Andrews, A. T., 1986, Electrophoresis: Theory, Techniques and Biochemical and Clinical Applications, (Clarenton, Oxford); Righetti, P. G., 1989, J. ofBiochem. Biophys.Methods 19, 1-20.

According to an embodiment, a separation medium suitable for the instant invention may be an entangled polymer solution.

For capillary electrophoresis, all separation media may be used. Non exhaustive examples of such media are recited in (Barbier V, Viovy JL. 2003. Advanced polymers for DNA separation. Curr Opin Biotechnol 14(1):51-7; Righetti, P. G. and C. Gelfi. 1997. In Analysis of Nucleic Acids by Capillary Electrophoresis, edited by C. Heller, Chromatographia, CE series 1, (Vieweg, Wiesbaden), pp. 255-271) ;

Also, some non polymeric matrices or separation media presenting sieving properties may be used (see for instance Rill, R., T. Liu, B. R. Locke and D. H. Van Winkle, 1998, Proc. Natl. Acad. Sci. USA 95,1534-1539)

For the performance of a separation, and thus for optimal operation of the invention, it may be however important to have a matrix with the higest discriminative power for size difference, and also for discrimination between heteroduplexes and homoduplexes to be privileged.

According to an embodiment, the separation medium according to the invention may comprises crosslinked or non-crosslinked polymers of acrylamide, methacrylamide or acrylamide derivatives.

In particular, sieving matrices based on PDMA (polydimethyl acrylamide), as described e.g. in US 5,567,292 to Madabhushi et al.), may be interesting within the scope of the invention.

For example, polymers convenient for the invention may include, but are not limited to N,N-disubstituted polyacrylamides, N-monosubstituted polyacrylamides, polymethacrylamide, polyvinylpyrrolidone, and the like. Exemplary substituents of the polyacrylamides includes $C_1$ to $C_{12}$ alkyl; halo-substituted $C_1$ to $C_{12}$ alkyl; methoxy-substituted $C_1$ to $C_{12}$ alkyl; hydroxyl-substituted $C_1$ to $C_{12}$ alkyl and the like.

Linear acrylamide may be a sieving polymer for DNA electrophoresis, well-known from those skilled on the art.

According to an embodiment, the separation medium suitable for the instant invention may comprise a block copolymer of acrylamide and dimethyl acrylamide.

The polymer may be a block copolymer(s) having an acrylamide backbone and polydimethylacrylamide (PDMA) side-chains or more generally block copolymer types, such as those cited in (Fr. Pat. Appl. 00/0856 to Barbier et al).

An analytical method of a step e) of a method according to the invention may be advantageously applied in non denaturating conditions.

However, additional components like denaturants may be included in a separation medium of the invention, such as denaturants to prevent the formation of duplexes or secondary structures in polynucleotides.

Denaturants may include formamide, e.g. 1-90%, urea e.g. 0.1-8 M, commercially or non-commercially available lactams, such as pyrrolidone, and the like. Guidance for their use in electrophoresis may be found in well known molecular biology references, e.g. Sambrook et al, Molecular Cloning: A Laboratory Manual, Second Edition (Cold Spring Harbor Laboratory, New York, 1989).

### Nucleotide base-pairing compound

According to an embodiment, the separation medium according to the invention may also comprise at least one compound able to undergo specific base pairing interaction in particular with a nucleotide involved in a mismatch.

Said compounds may contain one single base-pairing-unit, with "base pairing unit" meaning a molecular group able to undergo one base pairing interaction with one of the bases, A, T, G, U or C.

Such a compound may be used alone or in mixture with one or several other compounds able to undergo specific base pairing interactions with the base(s) of the nucleic acid(s) involved in the mismatch of a heteroduplex.

According to an embodiment, said compounds may be used at a combined concentration of at least 1 g/l of the separation medium used for contacting it or them with the nucleic acid to assay for mutation, preferably at least 10 g/l and most preferably at least 25 g/l.

By "combined concentration", it is understood the total concentration of said compounds, e.g. in the case of the use of several types of compounds, the combined concentration is the total of the concentrations of each compound. The concentration of such compound(s) is expressed with respect to the total volume of the medium containing the nucleic acid to analyze.

According to an embodiment, such a compound may be selected in the group consisting of an oligonucleotide having a length of less than 5 nucleotides, preferably less than 3 nucleotides and more preferably less than 2 nucleotides, a nucleoside, a base or a mixture thereof.

Non restrictive examples of such compounds are:

- the bases adenine (A), guanine (G), cytosine (C), uracile (U) and thymine (T),
- adenine, guanine, cytosine, uracile and thymine bearing various substitutions, and in particular substitutions having none effect on the amino and OH groups responsible for base pairing interactions in unsubstituted bases,
- the nucleosides formed with the bases A, T, G, C, U,
- the nucleotides formed with the bases A, T, G, C, U,
- oligonucleotide analogs, and variously substituted oligonucleotides, and
- the mixtures thereof.

According to an embodiment, said compound may be a nucleoside selected among the nucleosides adenosine, guanosine, uridine, cytidine, thymidine and mixtures thereof.

For instance, a method according to the invention may advantageously use cytidine and thymidine, or cytidine and adenosine, or guanosine and thymidine, or guanosine and adenosine, but not e.g. thymidine and adenosine, or cytidine and guanosine.

**<u>Signal</u>**

As stated previously, the analysis may be performed by comparing intensities and/or shapes of the signals issued from first nucleic acid fragment(s) to be analyzed, the second reference nucleic acid fragment, form(s) of first nucleic acid fragment(s) in which no large scale mutation involving said first nucleic acid fragment(s) is present and form of first nucleic acid fragment(s) equivalent to a non mutated form of said first nucleic acid fragment.

A shape of a signal may be characterized by a number of peaks of the signal, by a width of the signal, or both. A fragment with no mutation will usually lead to a single peak. By comparing the shape of a signal of a fragment to the shape of a signal of a control sample one can determine the presence or not of a mutation. The shape difference may be a difference in a number of peak (the maximum of peak should be 4 corresponding to the two homoduplexes and the two heteroduplexes). A peak shouldering or a peak widening may also be identified as a signature of the presence of a mutation. This shape differences may be determined by a visual observation by an operator or by a use of a specific curve analysis software (comparison of peak multiplicity and peak widening may easily be performed with curve analysis softwares, for example Peakfit , OriginPro (Originlab), or Igor (Wavemetrics)).

Preferably, however, an analysis may be performed by a dedicated automated software, able to analyse in a fully automated way a number of peaks in a signal, and a widening of the signal, and to compare the results, in particular a ratio of a signal width of the sample to a signal width of a reference signal, to a predefined value.

In contrast, the presence of large scale alterations may be detected by comparing the normalized peak intensity (or area) of a signal to a corresponding normalized peak intensity (or area) of a signal of a control sample without any large scale alteration. Peak intensity (or area) may be normalized by the peak intensity (or area) of an internal reference (fragment from a DNA region different from the gene of interest) present in the multiplex or by the peak intensity (or area) of another fragment of the gene of interest present in the sample during the separation.

A peak intensity (or area) higher than those of the control first nucleic acid fragment by a factor of at least 1.2, and preferable at least 1.35, may be a signature of the presence of a duplication.

A peak intensity (or area) at most 0.8, and preferably at most 0.7 times a peak intensity (area) of a control non-mutated first nucleic acid fragment by a factor of 0.5 ($\pm$ 0.3) may be the signature of the presence of a deletion.

These peak intensity (or area) differences may be determined by a visual observation by an operator or by a use of a specific curve analysis software.

"Peak intensity or signal intensity" is intended to mean a quantitative determination of a quantity of material corresponding to a given peak or signal. Typical ways of measuring the peak intensities may be measuring a maximum height as referred to the baseline, or an integrated area behind the signal.

Preferably, in particular if the signal contains several peaks, the intensity may be determined using the integrated area.

Different usual data treatment methods may be used within the invention, in order to improve the reliability of signal amplitude and signal shape analysis. Such methods may include, non exhaustively, baseline substraction, noise reduction, peak fitting with a predefined peak shape, spurious peaks elimination, and the like.

## POINT MUTATION

According to the invention, detectable point mutations include deletion mutation, insertion mutation, and substitution mutation wherein an incorrect base pairing occurs. Deletion and insertion mutations are also known as "frameshift" mutations, due to their effects on translation of the genetic code into proteins.

If the difference between two homologous strands of nucleic acid paired in a duplex form consists in a single nucleotide difference or a small insertion or deletion, a mismatched duplex may be formed. The methods according to the invention may be particularly efficient for detecting mismatched duplex.

More specifically, the method according to the invention may be particularly useful for the screening of a DNA fragment having a nucleic sequence related to a gene on which point mutation(s) has been associated or putatively associated with a disease or an increased predisposition to a disease. Said diseases may be different types of cancers, genetic diseases or increased predisposition to a disease such as, as an example, thalassemia, cardiovascular diseases, myopathy, cancer, and more generally genetically inheritable diseases. A non-exhaustive list of such diseases, with the associated genes, and prevalence in the population, is given in the following table as a matter of example. This list should not be considered by any means as limiting the scope of the invention, but is proposed here only to make it clear that the range of applications of the invention in human health is large and constantly expanding with the progress of genetics.

Non-limiting list of genes associated with increased predisposition to cancerous diseases, the diagnosis of which may constitute a privileged range of application of the invention.

| Predisposition | Associated putative mutated gene(s) | Frequency of mutation bearers in general population | Frequency of mutation bearers in cancer affected patients |
|---|---|---|---|
| Breast, ovary | BRCA1, BRCA2 | 1/500 | 1/30 |
| Colon, endometer (HNPCC syndrome) | hMLH1, hMSH2, hMSH6, hPMS2, TGFbeta | 1/500 | 1/20 |
| Melanoma | CDKN2A, CDK4 | 1/500 | 1/20 |
| Kidney | c-MET | 1/5000~10000 | 1/20 |
| Stomach (excluding HNPCC) | CDH1 | 1/10000~20000 | 1/100~200 |
| Colon | APC | 1/8000 | 1/100 |
| Hamartomatoses | | | |
| VHL | VHL | 1 /40000 | |
| NF2 | NF2 | 1 /30000 | |
| Peutz-Jegherz | LKB1 | 1/50000~100000 | |
| Gorlin syndrom | PTCH | 1/50000~100000 | |
| Cowden, Banayan-Zonana syndrom | PTEN | 1/50000~100000 | |
| NF1 | NF1 | 1/3000 | |
| Bourneville sclerosis | TSC1, TSC2 | 1/10000~15000 | |
| Multiple endocrinian neoplasia | | | |
| Type 1 | MEN1 | 1/30000~40000 | |

(continued)

| Predisposition | Associated putative mutated gene(s) | Frequency of mutation bearers in general population | Frequency of mutation bearers in cancer affected patients |
|---|---|---|---|
| Type 2 | Ret | 1/30000~40000 | 1/10~20 |
| Carney syndrom | PRKAR1A | 1/50000~80000 | |
| DNA breakage associated diseases | | | |
| Ataxia Telangiectasia | ATM | 1/40000~300000 | |
| Fanconi disease | 6 associated genes | 1/350000 | |
| Bloom disease | BLM | 1/1000000 | |
| Xeroderma pigmentosum | 8 associated genes | 1/500000~1000000 | |
| Werner disease | WRN | 1/300000~1000000 | |

In particular, the method according to the invention is particularly useful for the screening of the human breast cancer predisposing genes, (BRCA) like BRCA1 and BRCA2, for mutations.

According to a method of the present invention, alteration(s) of the wild type BRCA1 or BRCA2 locus may be detected. In addition, the methods may be performed by detecting the wild type BRCA1 or BRCA2 locus and thus confirming a lack of a predisposition to cancer at BRCA1 or BRCA2 locus.

## LARGE SCALE ALTERATION

According to the invention, this term is intended to encompass larger mutations than point mutation affecting multiple base pairs, that may consist in deletion or duplication.

In particular, some large scale alterations may arise due to "slippage" of the replication machinery in repeated DNA regions, leading to insertions or deletions of variable size.

Typically, the invention may be particularly useful in detecting large scale alterations, that may result in the presence of several amplifiable copies of a nucleic acid fragment on the genome or, in contrary, in the absence of amplification of a nucleic acid fragment. Typically such large scale alterations may involve very diverse nucleic acid lengths, sometimes as small as 10 or a few tens of kb, but more generally several hundred or several thousand bp.

Sometimes they may even involve an entire gene, thus covering several Mbp.

## NUCLEIC ACID AMPLIFICATION METHOD

According to an embodiment, the method according to the instant invention may comprise an additional step said step f) comprising at least an amplification and more particularly a semi-quantitative amplification, in non-saturating conditions, of first nucleic acid fragment(s) and second reference nucleic acid fragment, said step f) being performed before step b).

The most common nucleic acid amplification method is polymerase chain reaction (PCR), and this method may be a privileged method for performing the additional step f) in the invention.

Any linear or logarithmic method of amplification may be used, including, but not limited to, the ligase chain reaction, the polymerase chain reaction (PCR, RT-PCR) and techniques such as Rolling Circle Amplification (RCA) or the nucleic acid sequence based amplification (NASBA).

The polymerase chain reaction (PCR, RT-PCR) may be particularly convenient for the invention.

The amplification may be performed in non-saturating amplification conditions.

Within the invention, the expression "non-saturating amplification conditions" is intended to mean a number of amplification cycles, or more generally conditions of amplification, chosen so that the final number of copies of a nucleic acid fragment in the amplification product depends in a significant and reproducible way on the initial number of copies in the sample. If the amplification method is PCR, in particular, the number of cycles may be chosen to be in the "exponential domain", in which the final number of copies is approximately proportional to:

$$C_{final} = C_{initial} * 2^n$$

where n is the number of PCR cycles, and Cfinal and Cinitial are respectively the final and the initial number of copies of a given nucleic acid fragment.

The amplification may also be performed in the "linear domain", corresponding to the domain surrounding the inflexion point of the sigmoidal shape of the amplification curve (number of copies versus number of cycles).

Those skilled in the art know different ways to determine, for a given sample, the conditions suitables for satisfying the condition of having a final number of copies dependent on the initial number of copies.

For a genome of a given size, a simple way of determining those conditions may be to dilute the sample in order to have in the initial sample a given concentration of nucleic acid.

For example, as described in more detail in the examples below, for human diagnostic and thus for samples involving genomic human DNA, an initial DNA quantity of 100 ng may be suitable. For such a concentration, a number of 25 PCR cycles may be suitable.

A simple formula may also allow to extrapolate from this rule, if the quantity of DNA in the sample is different from this described above.

Typically, one can use the following formula:

$$\text{n optimal} = 2500/x.$$

in which x is the DNA quantity in the sample, expressed in ng.

This formula is valid for a volume of 10 $\mu$l. Different equivalent formulas may be determined by those knowledgeable in the art, for different sample volumes, different genomes, of different amplification conditions.

For instance, such a more general method may consist in performing in a first instance amplification in a quantitative PCR apparatus, such as the Light Cycler by Applera, and determining by this way the number of cycles "$n_i$" corresponding to the inflexion point of the amplification curve.

A number of amplification of a PCR or an RT-PCR cycles may range from $n_i$-7 to $n_i$+2, and preferably from $n_i$-3 to $n_i$+1, ni being a number of cycles corresponding to an inflexion or an amplification curve.

According to an embodiment, the PCR or RT-PCR may comprise a number of amplification ranging from 22 to 27.

At the end of a DNA amplification, the amplified nucleic acid may be renaturated in convenient conditions like for example by cooling down slowly in order to favor the formation of heteroduplexes, if a fragment is present in the sample with two different sequences, corresponding to different alleles.

Such conditions may essentially consist in heating the sample at a temperature around 90°C or higher, and cooling down this sample slowly (typically in the order of 1°C/mn). For instance, as described in examples, a temperature decay of 1°C/mn may be used.

## SAMPLE

A sample containing the nucleic acid fragment(s) to be analyzed may be a synthetic or natural sample, or a sample issued from biotechnology. In a privileged embodiment, it is a sample from a patient.

In particular, the invention encompasses all biological samples containing nucleic acid fragments without any particular limitation. More particularly, a biological sample according to the invention may originate from a cell, a tissue, an organ, a surgical or a biopsy specimen fixed or non-fixed such as bone marrow aspirates, or a biological fluid including body fluids such as whole blood, serum, plasma, cerebrospinal fluid, urine, lymph fluids, and various external secretions of the respiratory, intestinal and genito-urinary tracts, tears, saliva, milk, white blood cells, and cell culture supernatants. The origin of the sample can be animal (preferably mammal, more preferably human), plant, virus, bacteria, protozoan or fungus. The sample may be eukaryotic, prokaryotic, or acellular. Cells comprised in the biological sample, especially when coming from a tissue, organ, biological fluid or biopsy, may be cultivated in order to increase the number of available cells. The sample may contain cells from a single type or of mixed cell type. The cells, tissues and specimens may originate from normal individuals or from patient suffering from a disease or a disorder. The disease or disorder may be, for example, a cancer, a neurodegenerative disease, an inflammatory disease, a cardiovascular disease, an immune disorder, a body weight disorder such as obesity, etc. Any particular cell, cell type, pathological cell, cell at a particular state of development or disease progression, are contemplated in the present invention.

The method according to the invention may be particularly useful for either the diagnosis of the predisposition of diseases associated or putatively associated to specific point mutation(s) and/or large scale alteration(s) or the diagnosis or prognosis of such disease(s).

The instant invention is further related to the use of a method in accordance with the invention in the diagnosis of predisposition to genetic diseases or cancers or the diagnosis or prognosis of said diseases or cancers.

The invention also relates to the use of said methods in therapy of said diseases.

In particular, concerned diseases may include many cancers insofar as they are associated or putatively associated to specific point mutation(s) such as melanoma, ocular melanoma, leukemia, astrocytoma, glioblastoma, lymphoma, glioma, Hodgkin's lymphoma, multiple myeloma, sarcoma, myosarcoma, cholangiocarcinoma, squamous cell carcinoma, and cancers of the pancreas, breast, brain, prostate, bladder, thyroid, ovary, uterus, testis, kidney, stomach, colon and rectum.

The figures and examples given below are presented by way of non limiting illustration of the present invention.

## FIGURES

Figure 1 shows capillary-to-capillary reproducibility of area measurement of the different peaks obtained for example 1.

Figure 2 shows run to run reproducibility of mean normalized area measurement of the different peaks for example 2 on 5 successive runs

Figure 3 shows two superposed electrophoregramms corresponding to the separation of M1BC1-A and M1BC1-B stated in example 3.

Figure 4 shows two superposed electrophoregramms corresponding to the separation of M1BC1-A and M1BC1-C as stated in example 4.

Figure 5 shows the dispersion of normalized areas for fragments M1BC1-A, M1BC1-B and M1BC1-C as stated in example 5.

## EXAMPLES

All fragment used for separation are listed in Table 1. Type of substitution, fragment size, primer sequence and multiplex compositions are shown in the following table.

| Exon | Mutation | Fragment size | sens primer sequence | reverse primer sequence | Multiplex |
|---|---|---|---|---|---|
| 5 | | 290 | aatatctaaaagtagtattccaac | tgtatgaaacaaactcccac | M25BC2 |
| 21 | | 365 | gcagttatatagtttcttatcttta | atccctttttgagaaatgcagc | M25BC2 |
| 20 | | 412 | taatctcagcctcccaaagtt | aaaaagaataccctagatactaaa | M25BC2 |
| 16 | | 472 | tgtttttgtagtgaagattctag | tgcttaaccataatgcacttaaaa | M25BC2 |
| 19 | | 268 | aaggacctctcctctgtcat | tgtgcattgttaaggaaagtg | M1BC1-A |
| 6 | | 334 | agaggttttctactgttgctg | cagaactaaaattaacctagact | M1BC1-A |
| 22 | | 433 | gtggcaaattgacttaaaatcc | cagttctcaaatccttaccca | M1BC1-A |
| 11.5 | c. 2430T>C/Leu771 Leu | 519 | atactttcccagagctgaagt | tggcgctttgaaaccttgaat | M1BC1-A |
| 19 | | 268 | aaggacctctcctctgtcat | tgtgcattgttaaggaaagtg | M1BC1-B |
| 6 | deletion 3-16 | 334 | agaggttttctactgttgctg | cagaactaaaattaacctagact | M1BC1-B |
| 22 | | 433 | gtggcaaattgacttaaaatcc | cagttctcaaatccttaccca | M1BC1-B |
| 11.5 | deletion 3-16 | 519 | atactttcccagagctgaagt | tggcgctttgaaaccttgaat | M1BC1-B |
| 19 | duplication 18-19 | 268 | aaggacctctcctctgtcat | tgtgcattgttaaggaaagtg | M1BC1-C |
| 6 | | 334 | agaggttttctactgttgctg | cagaactaaaattaacctagact | M1BC1-C |
| 22 | | 433 | gtggcaaattgacttaaaatcc | cagttctcaaatccttaccca | M1BC1-C |
| 11.5 | c. 2430T>C/Leu771 Leu | 519 | atactttcccagagctgaagt | tggcgctttgaaaccttgaat | M1BC1-C |

PCR were done according the following protocol:

Samples used for mutation scanning were generated in 50 $\mu$L reaction volume containing 100 ng of genomic DNA, 0,06 units/$\mu$L of Taq polymerase (Amplitaq gold, Applied biosystems), 4 mM $MgCl_2$ 800 $\mu$M dNTPs and 1 x buffer (Applied Biosystems). Oligonucleotides concentrations were adapted in order to have a homogeneous intensity for all fragments contained in the multiplex.

PCR programs were run in a Icycler thermocycler (Biorad) and consisted in a first denaturation step of 96°C for 15 min followed by 25 cycles of denaturation at 96 °C for 30 s, annealing at 58 °C for 30 s, and extension at 72 °C for 30 s. PCR products were then denatured for 5 minutes at 96 °C and then gradually reannealed over 71 minutes by decreasing sample temperatures from 96°C to 25°C at 1°C/minute.

All experiments are performed in an ABI 3100 (Applied Biosystems, Foster city, USA) with 50 $\mu$m inner diameter capillaries with 50 cm effective length. Separation matrix is composed of Poly(acrylamide-g-poly(dimethylacrylamide)) at 5% (g/100mL) dissolved in Tris (50mM), Taps (50 mM), EDTA (2 mM) buffer. 2,5% of thymidine and 2,5% of cytidine are added to the matrix. Temperature is constant at 30°C. Finally SYBRgreen I (Molecular probes) is used at 1x for DNA detection.

Poly(acrylamide-g-poly(dimethylacrylamide)) characteristics are as following: copolymer molecular weight of 2 377 000 Da, polydimethylacrylamide graft molecular weight of 50 000 Da and polydimethylacrylamide graft mass density of 9.7%.

The copolymers of liquid separating medium were prepared according to the process of preparation disclosed in WO 02/01218. They have good sieving properties, and are surface-active.

## Example 1

Semi-quantitative measurement of DNA concentration using capillary electrophoresis with SYBRgreen I - Capillary-to-capillary variability.

Multiplex M25BC2 is used for this experiment. It is composed of 4 fragment differing by sizes (290 bp, 365 bp, 412 bp, and 472 bp) corresponding to exons 5, 21, 20 and 16 of gene BRCA2, respectively.

Injection is done at 2 kV for 20 s. Separations are performed during the same run in the same capillary array.

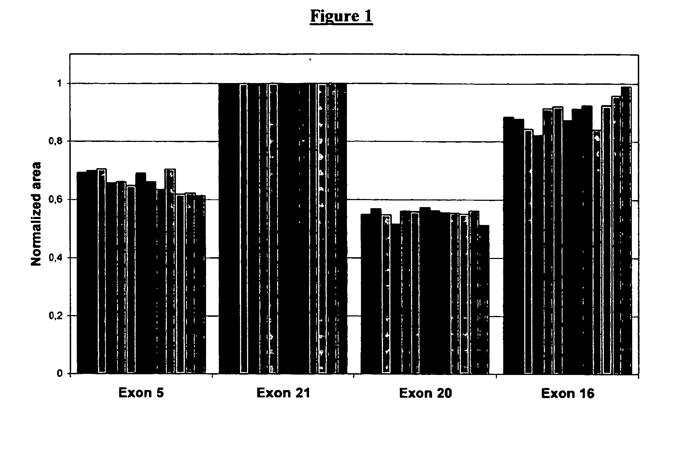

Figure 1 shows capillary-to-capillary reproducibility of area measurement of the different peaks obtained for example 1. All peak areas are normalized with area of the 2nd fragment (exon 21). Maximum observed variability are of the order of 10% which is perfectly compatible with large rearrangement detection. Indeed, the signature of a duplication would lead to an increase in the amplitude of at least 50%, and a deletion would lead to a decrease of the amplitude of around 50%. Since the error bar is of order 10%, these data show that there is no risk of false positives or false negatives in the analysis.

## Example 2

Semi-quantitative measurement of DNA concentration using capillary electrophoresis with SYBRgreen I - Run to run variability.

Multiplex M25BC2 is used for this set of experiment. It is composed of 4 fragments differing by sizes (290 bp, 365 bp, 412 bp, and 472 bp) corresponding to exons 5, 21, 20 and 16 of gene BRCA2, respectively.

Injection is done at 2 kV for 20 s. Separations are performed successively. 5 injections are performed.

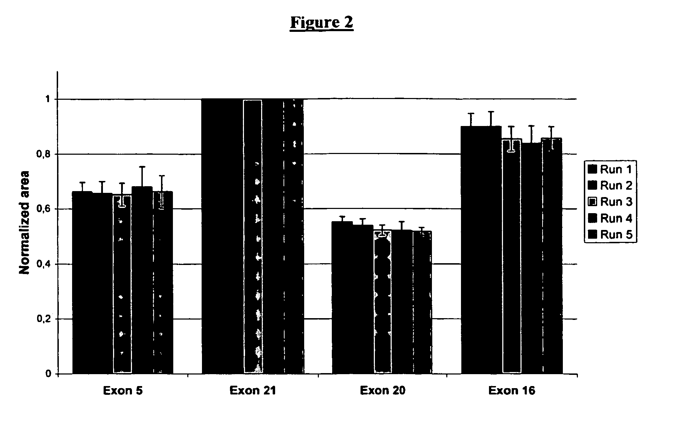

Figure 2 shows run to run reproducibility of mean normalized area measurement of the different peaks for example 2 on 5 successive runs. Peak areas are normalized with area of the 2nd fragment (exon 21). Mean normalized area is very reproducible from run to run. Error bars correspond to standard deviation. Again, this shows that false positives for the large scale mutations are very improbable.

## Example 3

Simultaneous detection of a large deletion in one patient and a substitution in a different patient.

Multiplex M1BC1 is used for this experiment. It is composed of 4 fragments differing by sizes (268 bp, 334 bp, 433 bp, and 519 bp) corresponding to different exons of BRCA1 gene (exon 19, exon 6, exon 22, and exon 11.5 respectively)

Multiplex M1BC1-A corresponds to a patient with no large chromosomic rearrangement but a single base substitution in exon 11.5.

Multiplex M1BC1-B corresponds to a patient with a large deletion of exon 3 to exon 16.

One can see that peaks corresponding to exon 6 and exon 11.5 of M1BC1-B are roughly 2 times smaller than those of M1BC1-A. This indicates that exon 6 and 11.5 are deleted in M1BC1-B. One can also notice that exon 11.5 of M1BC1-A has two peaks whereas exon 11.5 of M1BC1-B has a single peak. This indicates the presence of a small variation in exon 11.5 of M1BC1-A. Sequencing of this fragment revealed a single base substitution (c. 2430T>C/Leu771Leu).

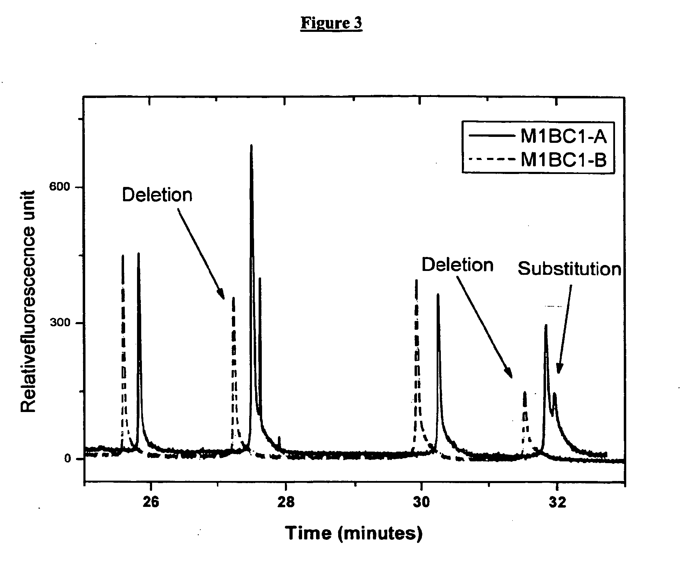

Figure 3 shows two superposed electrophoregramms corresponding to the separation of M1BC1-A and M1BC1-B stated in example 3.

### Example 4

Simultaneous detection of a large duplication and a single base substitution in one patient.

Multiplex M1BC1 is used for this experiment. It is composed of 4 fragments differing by sizes (268 bp, 334 bp, 433 bp, and 519 bp) corresponding to different exons of BRCA1 gene (exon 19, exon 6, exon 22, and exon 11.5 respectively)

Multiplex M1BC1-A corresponds to a patient with no large chromosomic rearrangement but a single base substitution in exon 11.5.

Multiplex M1BC1-C corresponds to a patient with a large duplication of exon 18 and 19 and a single base substitution in exon 11.5.

As in the previous example, one can observed the double peak of exon 11.5 of M1BC1-A corresponding to a single base substitution.

First peak of M1BC1-C is roughly 1.5 times higher than those of M1BC1-A which indicates a duplication of exon 19 in M1BC1-C. The same double peak as observed in M1BC1-A is present in exon 11.5 of M1BC1-C indicating the presence of a single base substitution.

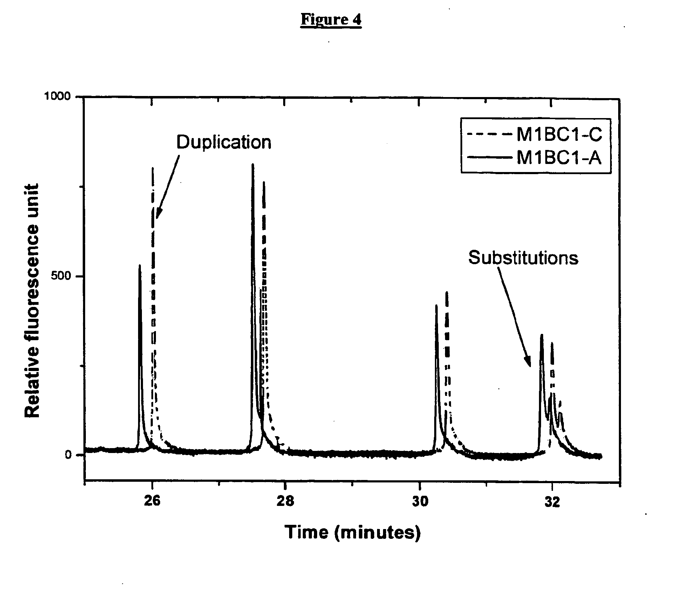

Figure 4 shows two superposed electrophoregramms corresponding to the separation of M1BC1-A and M1BC1-C as stated in example 4.

### Example 5

Dispersion of normalized areas for the detection of large rearrangement present in M1BC1-B and M1BC1-C.

6 separations with M1BC1-A were performed, 9 separations with M1BC1-B and 10 separations with M1BC1-C. Peak areas are normalized with the 3rd peak (exon 22) that do not present any kind of mutation in either of the multiplexes.

Deletions that correspond to a peak area difference of a factor 2 as compared to non-mutated fragments are very well identified (exon 6 and 11.5 of M1BC1-B). Duplication that corresponds to a peak area difference of a factor 1.5 as compared to non-mutated fragments present a very small overlap with non mutated fragments on the example of exon 19.

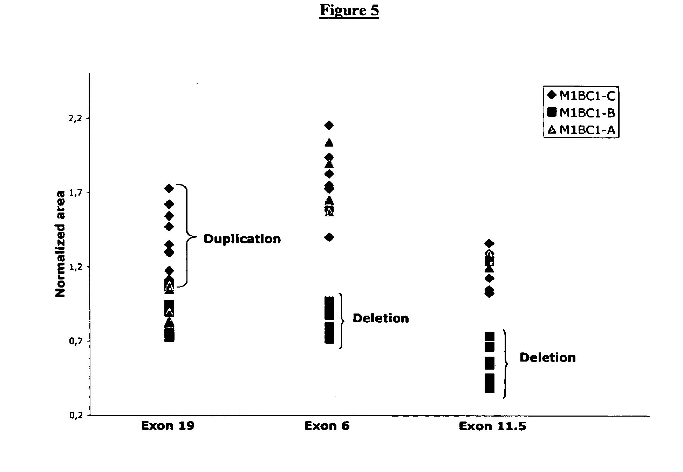

Figure 5 shows the dispersion of normalized areas for fragments M1BC1-A, M1BC1-B and M1BC1-C as stated in example 5.

### Claims

1. A method for detecting whether point mutation(s) and large scale alteration(s) are present in at least one nucleic acid fragment said "first nucleic acid fragment(s)", comprising at least the steps consisting in:

   a/ providing a sample liable to contain said first nucleic acid fragment and at least a second nucleic acid fragment distinguishable from the first nucleic acid fragment and acting as a quantitative reference,
   b/ denaturating said nucleic acid fragments and reannealing them in conditions suitable for obtaining a product containing homoduplexes and possible heteroduplexes,
   c/ conducting on said reanneled product an analytical method suitable for obtaining at least signal(s) discriminating existing duplex form(s) of the first nucleic acid fragment and relative quantitative data on said first nucleic acid fragment by comparing the intensity of its signal to the intensity of the signal obtained from the second reference nucleic acid fragment, and
   d/ comparing the relative quantitative data obtained in step c) to a control relative quantitative data expected for a first nucleic acid fragment in which no large scale alteration is present.

2. The method according to claim 1, comprising an additional step e) involving an analysis of the shape of the signal (s) from the first nucleic fragment(s) obtained in step c), said shape being **characterized by** a number of peaks of said signal, by a width of said signal, by a width of peak of said signal and/or by a shouldering of peak of said signal.

3. The method according to claim 2, wherein the additional step e) is performed by comparing the shape of the signal (s) from the first nucleic fragment(s) to the shape of the signal obtained by applying steps a) to c) to a form of said first nucleic acid fragments being equivalent to a non-mutated form of said first nucleic acid fragment(s).

4. The method according to any one of the preceding claims, said sample comprising more than 1 first nucleic acid fragment, in particular more than 2 and more particularly more than 5 first nucleic acid fragments.

5. The method according to any one of the preceding claims, wherein at least some or all of said first and second nucleic acid fragments are differing from each other by at least their respective length.

6. The method according to claim 5 wherein the length difference between fragments of different lengths is at least 1, in particular at least 3, in particular by at least 5, in particular by at least 10, in particular by at least 15, in particular by at least 20, and more particularly by at least 30 bases.

7. The method according to any one of the preceding claims wherein some or all first and second nucleic acid fragments are made distinguishable from each other(s) by at least distinct markers.

8. The method according to anyone of the preceding claims, wherein the second reference nucleic acid fragment is naturally present in the sample liable to contain said first nucleic acid fragment.

9. The method according to the preceding claim, wherein the second reference nucleic acid fragment and the first nucleic acid fragment(s) are derived from two distinct genes.

10. The method according to the claim 8, wherein the second reference nucleic acid fragment and the first nucleic acid fragment(s) are derived from the same gene.

11. The method according to anyone of the claims 1 to 7 wherein the second reference nucleic acid fragment is incorporated, into the sample liable to contain said first nucleic acid fragment, at a quantity predefined in regard of the total quantity of nucleic acid present in said sample.

12. The method according to anyone of the preceding claims, further comprising an additional step said step f) comprising at least an amplification, and more particularly a semi-quantitative amplification, in non-saturating conditions, of first nucleic acid fragment(s) and second reference nucleic acid fragment, said step f) being performed before step b).

13. The method according to claim 12 wherein step f) is performed by PCR or RT-PCR.

14. The method according to claim 12 or 13, wherein the PCR or RT-PCR comprises a number of amplification cycles ranging from 22 to 27.

15. The method according to claims 12 or 14, wherein the PCR or RT-PCR comprises a number of amplification cycles ranging from $n_i$ -7 to $n_i$ +2, and preferably from $n_i$ -3 to $n_i$ +1, $n_i$ being a number of cycles corresponding to an inflexion of an amplification curve.

16. The method according to claim 12, wherein said step f) is performed by rolling circle amplification or NASBA.

17. The method according to anyone of preceding claims, wherein said step c) is performed in non denaturating conditions.

18. The method according to anyone of preceding claims, wherein the separating analytical method is selected among an electrophoretic, chromatographic or a mass spectrometric method .

19. The method according to claim 18, wherein the separating analytical method is a capillary or multicapillary electrophoresis.

20. The method according to the preceding claim, wherein the electrophoresis analysis comprises the use of a separation medium.

21. The method according to the preceding claim, wherein the separation medium is an entangled polymer solution.

22. The method according to claims 18 or 19, wherein the separation medium comprises crosslinked or non-crosslinked polymers of acrylamide, methacrylamide or acrylamide derivatives.

23. The method according to anyone of claims 20 to 22, wherein the separation medium comprises a block copolymer of acrylamide and dimethyl acrylamide.

24. The method according to anyone of claims 20 to 23, wherein the separation medium further comprises at least one compound able to undergo a specific base pairing interaction with one of nucleotides A, T, G, C, said compound being at a concentration of at least 1g/l in said separation medium.

25. The method according to anyone of preceding claims, wherein steps c), d) and/ or e) are performed by an automated software.

26. The method for the diagnostic of a predisposition to cancer, comprising a method as defined according to anyone of claim 1 to 25.

27. A method for cancer diagnosis, comprising a method as defined according to anyone of claim 1 to 25.

28. A method for the diagnostic of a genetic disease, comprising a method as defined according to anyone of claim 1 to 25.

29. A method for discovering new targets for therapy or screening efficiency of a therapy, comprising a method as defined according to anyone of claim 1 to 25.

30. A method for discovering new biomarkers for a disease or a pathological condition, comprising a method as defined according to anyone of claims 1 to 25.


**Patentansprüche**

1. Verfahren zum Erfassen, ob (eine) Punktmutation(en) und (eine) große Veränderung(en) in mindestens einem Nukleinsäurefragment, dem "ersten Nukleinsäurefragment"/den "ersten Nukleinsäurefragementen", vorhanden sind, welches zumindest die folgenden Schritte aufweist:

   a) es wird eine Probe bereitgestellt, die das erste Nukleinsäurefragment und mindestens ein von dem ersten Nukleinsäurefragment unterscheidbares und als quantitative Referenz dienendes zweites Nukleinsäurefragment enthält,
   b) die Nukleinsäurefragmente werden denaturiert und unter solchen Bedingungen reassoziiert, dass ein Produkt gewonnen werden kann, das Homoduplexe und mögliche Heteroduplexe enthält,
   c) an dem reassoziierten Produkt wird ein analytisches Verfahren durchgeführt, das geeignet ist, ein Signal/ Signale zu gewinnen, das/die eine bestehende Duplexform/bestehende Duplexformen des ersten Nukeinsäurefragments und relative quantitative Daten des ersten Nukleinsäurefragments unterscheidet, indem die Intensität ihres Signals mit der Intensität des vom zweiten Referenz-Nukleinsäurefragment erhaltenen Signals verglichen wird, und
   d) die in Schritt c) erhaltenen relativen quantitativen Daten werden mit relativen quantitativen Kontrolldaten verglichen, die für ein erstes Nukleinsäurefragment erwartet werden, in dem keine große Veränderung vorhanden ist.

2. Verfahren nach Anspruch 1, mit einem zusätzlichen Schritt e), der eine Formanalyse des Signals/der Signale umfasst, die im Schritt c) von dem ersten Nukleinfragment/den ersten Nukleinfragementen erhalten wird, wobei die Form durch eine Anzahl von Spitzen des Signals, durch eine Breite des Signals, durch eine Spitzenbreite des Signals und/oder durch eine Schulter einer Spitze des Signals charakterisiert ist.

3. Verfahren nach Anspruch 2, wobei der zusätzliche Schritt e) durch Vergleichen der Form des Signals/der Signale des ersten Nukleinfragment/der, ersten Nukleinfragmente mit der Form des Signals durchgeführt wird, das durch Anwenden der Schritte a) bis c) auf eine Form der ersten Nukleinsäurefragmente, die zu einer nicht mutierten Form des ersten Nukleinsäurefragments/der ersten Nukleinsäurefragmente äquivalent sind, erhalten wird.

4. Verfahren nach einem der vorhergehenden Ansprüche, wobei die Probe mehr als ein erstes Nukleinsäurefragment, insbesondere mehr als zwei und ganz besonders mehr als fünf erste Nukleinsäurefragmente, umfasst.

5. Verfahren nach einem der vorhergehenden Ansprüche, wobei mindestens einige oder alle der ersten und zweiten Nukleinsäurefragmente sich mindestens durch ihre jeweilige Länge voneinander unterscheiden,

6. Verfahren nach Anspruch 5, wobei der Längenunterschied zwischen Fragmenten unterschiedlicher Länge mindestens 1, insbesondere mindestens 3, insbesondere mindestens 5, insbesondere mindestens 10, insbesondere mindestens 15, insbesondere mindestens 20, und ganz besonders mindestens 30 Basen beträgt.

7. Verfahren nach einem der vorhergehenden Ansprüche, wobei einige oder alle ersten und zweiten Nukleinsäurefragmente mindestens durch unterschiedliche Marker voneinander unterscheidbar gemacht sind.

8. Verfahren nach einem der vorhergehenden Ansprüche, wobei das zweite Referenz-Nukleinsäurefragment in der Probe, die das erste Nukleinsäurefragment enthält, natürlich vorkommt

9. Verfahren nach dem vorhergehenden Anspruch, wobei das zweite Referenz-Nukleinsäurefragment und das erste Nukleinsäurefragment/die ersten Nukleinsäurefragmente von zwei unterschiedlichen Genen abgeleitet sind.

10. Verfahren nach Anspruch 8, wobei das zweite Referenz-Nukleinsäurefragment und das erste Nukleinsäurefragment/die ersten Nukleinsäurefragmente von dem selben Gen abgeleitet sind.

11. Verfahren nach einem der Ansprüche 1 bis 7, wobei das zweite Referenz-Nukleinsäurefragment in der Probe, die das erste Nukleinsäurefragment enthält, in einer vorgegebenen Menge bezüglich der Gesamtmenge der in der Probe vorhandenen Nukleinsäure einbezogen ist.

12. Verfahren nach einem der vorhergehenden Ansprüche, ferner mit einem zusätzlichen Schritt f), der mindestens eine Amplifikation, und insbesondere eine semi-quantitative Amplifikation, des ersten Nukleinsäurefragments/der ersten Nukleinsäurefragmente und des zweiten Referenz-Nukleinsäurefragments unter Nichtsättigungsbedingungen umfasst, wobei der Schritt f) vor Schritt b) durchgeführt wird.

13. Verfahren nach Anspruch 12, wobei Schritt f) mittels PCR oder RT-PCR durchgeführt wird.

14. Verfahren nach Anspruch 12 oder 13, wobei die PCR oder die RT-PCR eine Anzahl von 22 bis 27 Amplifikationszyklen umfasst.

15. Verfahren nach Anspruch 12 oder 14, wobei die PCR oder die RT-PCR eine Amplifikations-Zyklenanzahl von $n_i$ -7 bis $n_i$ +2, vorzugsweise von $n_i$ -3 bis $n_i$ +1, umfasst, wobei $n_i$ eine Anzahl von Zyklen ist, die einem Wendepunkt einer Amplifikationskurve entspricht.

16. Verfahren nach Anspruch 12, wobei der Schritt f) durch Rolling Circle Amplifikation oder NASBA durchgeführt wird.

17. Verfahren nach einem der vorhergehenden Ansprüche, wobei Schritt c) unter nicht denaturierenden Bedingungen durchgeführt wird.

18. Verfahren nach einem der vorhergehenden Ansprüche, wobei das analytische Trennverfahren ein elektrophoretisches, chromatographisches oder ein massenspektrometrisches Verfahren ist.

19. Verfahren nach Anspruch 18, wobei das analytische Trennverfahren eine kapillare oder multikapillare Elektrophorese ist.

20. Verfahren nach dem vorhergehenden Anspruch, wobei die Elektrophoreseanalyse die Verwendung eines Trennmediums beinhaltet.

21. Verfahren nach dem vorhergehenden Anspruch, wobei das Trennmedium eine Polymernetzwerklösung ist.

22. Verfahren nach Anspruch 18 oder 19, wobei das Trennmedium vernetzte oder nicht vernetzte Polymere von Acrylamid, Methacrylamid oder Acrylamidderivaten enthält.

**23.** Verfahren nach einem der Ansprüche 20 bis 22, wobei das Trennmedium ein Blockcopolymer von Acrylamid und Dimethylacrylamid enthält.

**24.** Verfahren nach einem der Ansprüche 20 bis 23, wobei das Trennmedium ferner mindestens eine Verbindung enthält, die geeignet ist, eine spezifische Basenpaarwechselwirkung mit einem der Nukleotide A, T, G, C einzugehen, wobei die Verbindung in einer Konzentration von mindestens 1g/l in dem Trennmedium vorliegt.

**25.** Verfahren nach einem der vorhergehenden Ansprüche, wobei Schritte c), d) und/oder e) durch eine automatisierte Software durchgeführt werden.

**26.** Verfahren zur Diagnose einer Krebsprädisposition, das ein in Ansprüchen 1 bis 25 definiertes Verfahren beinhaltet

**27.** Verfahren zur Krebsdiagnose, das ein in Ansprüchen 1 bis 25 definiertes Verfahren beinhaltet.

**28.** Verfahren zur Diagnose einer Erbkrankheit, das ein in Ansprüchen 1 bis 25 definiertes Verfahren beinhaltet.

**29.** Verfahren zum Auffinden neuer Zielmoleküle für die Therapie oder für die Screeningeffizienz einer Therapie, das ein in Ansprüchen 1 bis 25 definiertes Verfahren beinhaltet.

**30.** Verfahren zum Auffinden neuer Biomarker für eine Krankheit oder für einen pathologischen Zustand, das ein in Ansprüchen 1 bis 25 definiertes Verfahren beinhaltet.

**Revendications**

**1.** Procédé pour détecter si une (des) mutations ponctuelle(s) et une (des) altération(s) à grande échelle sont présentes dans au moins un fragment d'acide nucléique, dit(s) "premier(s) fragment(s) d'acide nucléique", comprenant au moins les étapes consistant à :

a/ fournir un échantillon susceptible de contenir ledit premier fragment d'acide nucléique et au moins un second fragment d'acide nucléique différenciable du premier fragment d'acide nucléique et agissant en tant que référence quantitative,
b/ dénaturer lesdits fragments d'acide nucléique et les rehybrider dans des conditions convenant à l'obtention d'un produit contenant des homoduplex et des hétéroduplex éventuels,
c/ effectuer, sur ledit produit rehybridé, un traitement analytique convenant pour obtenir au moins un (des) signal (signaux) différenciant la (les) forme(s) de duplex existante(s) du premier fragment d'acide nucléique et des données quantitatives relatives sur ledit premier fragment d'acide nucléique en comparant l'intensité de son signal à l'intensité du signal obtenu à partir du second fragment d'acide nucléique de référence, et
d/ comparer les données quantitatives relatives obtenues dans l'étape c) à des données quantitatives relatives témoins prévues pour un premier fragment d'acide nucléique dans lequel une altération à grande échelle n'est pas présente.

**2.** Procédé selon la revendication 1, comprenant une étape supplémentaire e) faisant intervenir une analyse de la forme du (des) signal (signaux) du (des) premier(s) fragment(s) d'acide nucléique obtenu(s) dans l'étape c), ladite forme étant **caractérisée par** un nombre de pics dudit signal, par une largeur dudit signal, par une largeur de pic dudit signal et/ou par un épaulement de pic dudit signal.

**3.** Procédé selon la revendication 2, dans lequel l'étape supplémentaire e) est effectuée en comparant la forme du (des) signal (signaux) du (des) premier(s) fragment(s) d'acide nucléique à la forme du signal obtenu en mettant en oeuvre les étapes a) à c) à une forme desdits premiers fragments d'acide nucléique qui sont équivalents à une forme non mutée dudit (desdits) premier(s) fragment(s) d'acide nucléique.

**4.** Procédé selon l'une quelconque des revendications précédentes, ledit échantillon comprenant plus de 1 premier fragment d'acide nucléique, en particulier plus de 2 et plus particulièrement plus de 5 fragments d'acide nucléique.

**5.** Procédé selon l'une quelconque des revendications précédentes, dans lequel au moins certains, ou tous, desdits premiers et seconds fragments d'acide nucléique diffèrent entre eux au moins par leur longueur respective.

**6.** Procédé selon la revendication 5, dans lequel la différence de longueur entre les fragments de longueurs différentes est d'au moins 1, en particulier d'au moins 3, en particulier d'au moins 5, en particulier d'au moins 10, en particulier d'au moins 15, en particulier d'au moins 20 et plus particulièrement d'au moins 30 bases.

**7.** Procédé selon l'une quelconque des revendications précédentes, dans lequel certains, ou tous, des premiers et seconds fragments d'acide nucléique sont rendus différenciables entre eux par au moins des marqueurs distincts.

**8.** Procédé selon l'une quelconque des revendications précédentes, dans lequel le second fragment d'acide nucléique de référence est naturellement présent dans l'échantillon susceptible de contenir ledit premier fragment d'acide nucléique.

**9.** Procédé selon la revendication précédente, dans lequel le second fragment d'acide nucléique de référence et le(s) premier(s) fragment(s) d'acide nucléique sont dérivés de deux gènes distincts.

**10.** Procédé selon la revendication 8, dans lequel le second fragment d'acide nucléique de référence et le(s) premier(s) fragment(s) d'acide nucléique sont dérivés du même gène.

**11.** Procédé selon l'une quelconque des revendications 1 à 7, dans lequel le second fragment d'acide nucléique de référence est incorporé dans l'échantillon susceptible de contenir ledit premier fragment d'acide nucléique, en une quantité prédéfinie en fonction de la quantité totale d'acide nucléique présente dans ledit échantillon.

**12.** Procédé selon l'une quelconque des revendications précédentes, comprenant en outre une étape supplémentaire, ladite étape f), comprenant au moins une amplification, et plus particulièrement une amplification semi-quantitative, dans des conditions non saturantes, du (des) premier(s) fragment(s) d'acide nucléique et du second fragment d'acide nucléique de référence, ladite étape f) étant effectuée avant l'étape b).

**13.** Procédé selon la revendication 12, dans lequel l'étape f) est effectuée par PCR ou RT-PCR.

**14.** Procédé selon la revendication 12 ou 13, dans lequel la PCR ou RT-PCR comprend un certain nombre de cycles d'amplification allant de 22 à 27.

**15.** Procédé selon les revendications 12 ou 14, dans lequel la PCR ou RT-PCR comprend un certain nombre de cycles d'amplification allant de $n_i$ -7 à $n_i$ +2, et de préférence de $n_i$ -3 à $n_i$ +1, $n_i$ étant un nombre de cycles correspondant à une inflexion d'une courbe d'amplification.

**16.** Procédé selon la revendication 12, dans lequel ladite étape f) est effectuée par amplification par cercle roulant ou NASBA.

**17.** Procédé selon l'une quelconque des revendications précédentes, dans lequel ladite étape c) est effectuée dans des conditions non dénaturantes.

**18.** Procédé selon l'une quelconque des revendications précédentes, dans lequel le procédé analytique de séparation est choisi parmi un procédé électrophorétique; chromatographique ou spectrométrique de masse.

**19.** Procédé selon la revendication 18, dans lequel le procédé analytique de séparation est une électrophorèse capillaire ou multicapillaire.

**20.** Procédé selon la revendication précédente, dans lequel l'analyse électrophorétique comprend l'utilisation d'un milieu de séparation.

**21.** Procédé selon la revendication précédente, dans lequel le milieu de séparation est une solution de polymère enchevêtré.

**22.** Procédé selon les revendications 18 ou 19, dans lequel le milieu de séparation comprend des polymères réticulés ou non réticulés d'acrylamide, de méthacrylamide ou de dérivés d'acrylamide.

**23.** Procédé selon l'une quelconque des revendications 20 à 22, dans lequel le milieu de séparation comprend un copolymère séquencé d'acrylamide et de diméthylacrylamide

**24.** Procédé selon l'une quelconque des revendications 20 à 23, dans lequel le milieu de séparation comprend en outre au moins un composé apte à subir une interaction d'appariement de bases spécifique avec l'un des nucléotides A, T, G, C, ledit composé étant à une concentration d'au moins 1 g/l dans ledit milieu de séparation.

**25.** Procédé selon l'une quelconque des revendications précédentes, dans lequel les étapes c), d) et/ou e) sont effectuées par un logiciel automatisé.

**26.** Procédé pour le diagnostic d'une prédisposition à un cancer, comprenant un procédé selon l'une quelconque des revendications 1 à 25.

**27.** Procédé pour diagnostiquer un cancer, comprenant un procédé selon l'une quelconque des revendications 1 à 25.

**28.** Procédé pour diagnostiquer une maladie génétique, comprenant un procédé selon l'une quelconque des revendications 1 à 25.

**29.** Procédé pour découvrir de nouvelles cibles pour une thérapie ou déterminer l'efficacité d'une thérapie, comprenant un procédé selon l'une quelconque des revendications 1 à 25.

**30.** Procédé pour découvrir de nouveaux biomarqueurs pour une maladie ou une affection pathologique, comprenant un procédé selon l'une quelconque des revendications 1 à 25.

## Figure 1

## Figure 2

**Figure 3**

## Figure 4

**Figure 5**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 0134839 A
- US 5567292 A, Madabhushi
- WO 0201218 A


### Non-patent literature cited in the description

- **WAGNER, T ; STOPPA-LYONNET, D ; FLEISCHMANN,E. ; MUHR, D ; PAGES, S ; SANDBERG, T ; CAUX, V ; MOESLINGER, R ; LANGBAUER, G ; BORG, A.** *Genomics,* 1999, vol. 62, 369-376
- **ORITA M ; IWAHANA H ; KANAZAWA H ; HAYASHI K ; SEKIYA T.** Detection of polymorphisms of human DNA by gel electrophoresis as single-strand conformation polymorphisms. *Proc Natl Acad Sci U S A,* 1989, vol. 86 (8), 2766-70
- **COTTON RG ; RODRIGUES NR ; CAMPBELL RD.** Reactivity of cytosine and thymine in single-base-pair mismatches with hydroxylamine and osmium tetroxide and its application to the study of mutations. *Proc Natl Acad Sci U S A,* 1988, vol. 85 (12), 4397-401
- **SCHOUTEN JP ; MCELGUNN CJ ; WAAIJER R ; ZWIJNENBURG D ; DIEPVENS F ; PALS G.** Relative quantification of 40 nucleic acid sequences by multiplex ligation-dependent probe amplification. *Nucleic Acids Res,* 2002, vol. 30 (12), e57
- **CASILLI F ; DI ROCCO ZC ; GAD S ; TOURNIER I ; STOPPA-LYONNET D ; FREBOURG T ; TOSI M.** Rapid detection of novel BRCA1 rearrangements in high-risk breast-ovarian cancer families using multiplex PCR of short fluorescent fragments. *Hum Mutat,* 2002, vol. 20 (3), 218-26
- **DEHAINAULT C ; LAUGE A ; CAUX-MONCOUTIER V ; PAGES-BERHOUET S ; DOZ F ; DESJARDINS L ; COUTURIER J ; GAUTHIER-VILLARS M ; STOPPA-LYONNET D ; HOUDAYER C.** Multiplex PCR/liquid chromatography assay for detection of gene rearrangements: application to RB1 gene. *Nucleic Acids Res,* 2004, vol. 32 (18), e139
- **LAKEN SJ ; JACKSON PE ; KINZLER KW ; VOGELSTEIN B ; STRICKLAND PT ; GROOPMAN JD ; FRIESEN MD.** Genotyping by mass spectrometric analysis of short DNA fragments. *Nat Biotechnol,* 1998, vol. 16 (13), 1352-6
- **ANDREWS, A. T.** Electrophoresis: Theory, Techniques and Biochemical and Clinical Applications. Clarenton, 1986
- **RIGHETTI, P. G.** *J. ofBiochem. Biophys.Methods,* 1989, vol. 19, 1-20
- **BARBIER V ; VIOVY JL.** Advanced polymers for DNA separation. *Curr Opin Biotechnol,* 2003, vol. 14 (1), 51-7
- **RIGHETTI, P. G. ; C. GELFI.** Analysis of Nucleic Acids by Capillary Electrophoresis. Vieweg, 1997, 255-271
- **RILL, R. ; T. LIU ; B. R. LOCKE ; D. H. VAN WINKLE.** *Proc. Natl. Acad. Sci. USA,* 1998, vol. 95, 1534-1539
- **SAMBROOK et al.** Molecular Cloning: A Laboratory Manual. Cold Spring Harbor Laboratory, 1989